# FASCICULE DE BREVET EUROPEEN

(11) **EP 3 810 034 B1**
(45) Date de publication et mention de la délivrance du brevet: **16.10.2024**
(21) Numéro de dépôt: 19737004.2
(22) Date de dépôt: 21.06.2019
(51) Int. Cl.: A61F 2/12, A61L 27/36, A61L 27/50, A61F 2/00

(54) **MATRICE DERMIQUE ACELLULAIRE, NOTAMMENT POUR PROTHESE MAMMAIRE**
AZELLULÄRE DERMALE MATRIX, INSBESONDERE FÜR EINE BRUSTPROTHESE
ACELLULAR DERMAL MATRIX, IN PARTICULAR FOR A MAMMARY PROSTHESIS

(30) Priorité: 22.06.2018 FR 1855590
(43) Date de publication de la demande: 28.04.2021
(73) Titulaire: Meccellis Biotech, 17000 La Rochelle (FR)
(72) Inventeur: PERES, Anthony, 17139 DOMPIERRE SUR MER (FR); HOFMANSKI, Guillame, 61250 MIEUXCE (FR); LAMMELIN, Aurélie, 78550 RICHEBOURG (FR)
(74) Mandataire: Aquinov
(86) Numéro de dépôt international: PCT/EP2019/066522
(87) Numéro de publication internationale: WO 2019/243599

(56) Documents cités:
- WO-A1-2015/065923
- WO-A2-2009/009620
- US-A1- 2017 224 460
- US-A1- 2018 008 745

## Description

L'invention concerne une matrice dermique acellulaire, en particulier pour couvrir un implant mammaire en chirurgie du sein.

L'invention sera plus particulièrement décrite en regard d'une prothèse mammaire pour la reconstruction du sein, sans toutefois y être limitée. Elle s'applique à toute matrice dermique acellulaire destinée à couvrir une prothèse avec relief, notamment avec au moins une forme bombée.

Dans la reconstruction du sein après une mastectomie ou dans le cadre d'une autre opération chirurgicale de reconstitution esthétique du sein, il est à présent de plus en plus utilisé une matrice dermique acellulaire qui couvre l'implant/la prothèse et qui est relié au muscle du grand pectoral, évitant ainsi d'étirer préalablement la peau et les muscles avant la mise en place de l'implant. En outre, une telle matrice aide au soutien des tissus et à leur régénération.

Il est connu de la demande de brevet américain US2017/0224460 une matrice dermique acellulaire comportant une feuille souple produite par exemple à partir de tissu porcin, cette feuille comprenant une multitude de trous ronds traversants. Les trous permettent un écoulement de fluide et peuvent être utilisés pour la fixation de moyens de suture. WO2015/065923 A1 divulgue une matrice dermique acellulaire utile pour la reconstruction d'un sein, comprenant des rangées de fenestrations formées dans celle-ci.

L'invention a pour but de proposer une autre matrice dermique acellulaire perforée.

L'invention est définie par la revendication 1 annexée.

Selon l'invention, la matrice dermique acellulaire comporte une feuille pleine et souple qui est dotée d'une multiplicité d'ouvertures traversantes selon l'épaisseur de la feuille. Les ouvertures ont une forme allongée dans au moins une direction, en particulier forment des fentes au moins à l'état de repos de la matrice.

On entend par « état de repos de la matrice », l'état où elle ne subit aucun étirement.

La forme des ouvertures à l'état de repos de la matrice ou à l'état étiré, est oblongue.

Ainsi, la forme allongée des ouvertures permet un étirement dans toutes les directions de la feuille, ce qui permet à la matrice de parfaitement épouser la surface d'accueil de ladite matrice en position d'utilisation. La section des ouvertures est ainsi variable pour s'adapter aux différents efforts de traction, sans aucun risque de plis à la surface de la matrice ou de tensions non voulues. La configuration des ouvertures et zones pleines intermédiaires de la matrice permet une conformation optimale de la matrice sur l'implant (la surface d'accueil) et ce quelle que soit la taille.

Selon l'invention, les ouvertures qui passent de fentes à orifices en position étirée de la matrice restent de forme oblongue.

Selon une caractéristique, les ouvertures sont aptes à s'ouvrir/s'étendre/s'étirer (à l'état étiré de la matrice) selon une géométrie de section polygonale, en particulier en forme de losange. D'autres géométries sont envisageables telles qu'une forme en étoile à partir d'une découpe allongée selon au moins une direction.

Selon une autre caractéristique les ouvertures à l'état étiré de la matrice ont une section de grandeur variable. Cette grandeur est fonction du relief épousé par la matrice lorsqu'elle recouvre notamment une prothèse.

De préférence, les ouvertures (les fentes) ont toutes la même forme à l'état de repos de la matrice.

Les ouvertures sous la forme de fentes (au moins à l'état de repos de la matrice) présentent une longueur comprise entre 5 et 15 mm, de préférence de l'ordre de 10 mm. Une fente à l'état de repos de la matrice présente de préférence une largeur d'au plus 1 mm ; cette largeur correspond à la largeur du couteau de découpe.

Les ouvertures/fentes sont issues d'une découpe de la feuille, par des moyens de découpe appropriés, par exemple du type emporte-pièces, couteau, cutter.

La forme allongée des ouvertures/fentes à l'état de repos de la matrice est telle que le ratio d'expansion de la largeur des ouvertures est de préférence au moins de 2 pour 1 à l'état étiré de la matrice, notamment entre 2 :1 et 5 :1.

Selon l'invention, les ouvertures sont distribuées en colonnes, en particulier en étant séparées d'une distance comprise entre 2 et 5 mm, de préférence de l'ordre de 3 mm pour une même colonne et de l'ordre de 2 mm pour deux colonnes adjacentes.

Les ouvertures forment un maillage.

Selon une autre caractéristique, les ouvertures sont agencées sur la feuille dans une zone localisée, de préférence de manière centrale par rapport à l'une des dimensions de la feuille (en particulier de manière centrale par rapport à la largeur de la feuille lorsqu'elle est rectangulaire ou du moins lorsqu'elle est de forme allongée), et de préférence la feuille comporte des zones pleines au sein de la zone localisée des ouvertures, en particulier les zones pleines formant des bandes longitudinales régulièrement espacées, de préférence de largeur comprise entre 5 et 15 mm, de préférence sans s'étendre sur toute la longueur de la zone localisée lorsque ladite zone localisée est oblongue.

Avantageusement, la zone localisée dotée d'ouvertures comporte d'une part, des zones pleines longitudinales s'étendant en longueur dans la direction longitudinale de la feuille, et d'autre part, en d'autres endroits, des portions pleines, notamment de forme sensiblement triangulaire, qui sont disposées symétriquement en regard au niveau de la partie la plus large de la zone localisée selon une direction transversale aux zones pleines longitudinales De préférence, des portions pleines, notamment de forme sensiblement triangulaire, peuvent en outre être disposées symétriquement en regard au niveau de la partie la plus large de la zone localisée (et au-delà et de chaque côté des bandes longitudinales pleines) selon une direction transversale aux bandes pleines longitudinales.

Les ouvertures présentent un espacement régulier entre elles hormis dans des zones localisées qui sont pleines et ont des dimensions en longueur et en largeur qui sont bien plus grandes que l'espacement entre les ouvertures. Ces zones pleines localisées ont de préférence la forme de bandes longitudinales précitées ou des portions pleines triangulaires. Ainsi, ces zones pleines au sein de la zone localisée des ouvertures constituent des ruptures du motif régulier des ouvertures.

La feuille est de forme allongée selon une direction longitudinale et présente deux parties opposées selon un plan médian transversal à la direction longitudinale, la première partie est totalement pleine et la seconde partie est pleine et dotée des ouvertures dans une zone localisée.

Les deux parties présentent de préférence une forme de contour symétrique par rapport au plan médian transversal.

La feuille est de forme par exemple rectangulaire et destinée à être découpée au niveau de sa périphérie pour épouser les lignes courbes d'une prothèse lorsque la matrice enveloppera la prothèse dans son utilisation.

Les deux parties de la feuille sont chacune par exemple à contour courbe selon une forme ovoïde ou en forme de coquillage. Les deux parties à ligne périphérique courbe se rejoignent au niveau du plan médian en une section rétrécie.

La zone localisée dotée d'ouvertures est uniquement sur l'une des deux parties et centralisée par rapport à la périphérie de ladite partie.

Selon l'invention, les ouvertures sont agencées sur la feuille dans une zone localisée formant une surface ovoïde, la zone localisée dotée des ouvertures comportant des bandes pleines espacées s'étendant dans la direction longitudinale, de préférence en partant de l'extrémité la plus étroite de la surface ovoïde, de préférence sans s'étendre jusqu'à l'extrémité opposée, et de préférence des portions pleines, notamment de forme sensiblement triangulaire, étant disposées symétriquement en regard au niveau de la partie (transversale à la longueur) la plus large de la forme ovoïde.

L'invention porte également sur un ensemble comportant une matrice dermique acellulaire précitée de l'invention, et une prothèse, notamment mammaire, qui est recouverte en tout ou partie par la matrice, en particulier les ouvertures étant agencées dans une zone localisée qui correspond à une surface en relief de la prothèse.

La partie pleine de la feuille souple (en dehors donc de la zone localisée perforée) est de dimensions adaptées pour recouvrir tout ou partie de la prothèse. La zone localisée perforée présente une géométrie et des dimensions adaptées à la forme de la prothèse.

La présente invention est maintenant décrite à l'aide d'exemples uniquement illustratifs et nullement limitatifs de la portée de l'invention, et à partir des illustrations ci-jointes, dans lesquelles :
- La figure 1 représente une vue schématique de dessus d'un exemple de réalisation de la matrice dermique acellulaire selon l'invention, à l'état de repos ;
- La figure 2 représente la matrice de la figure 1 dans un état étiré ;
- Les figures 3 à 5 sont des vues en perspective de différentes étapes successives d'association de la matrice à une prothèse mammaire ;
- La figure 6 est une vue de l'ensemble de la matrice enveloppant la prothèse, l'ensemble étant prêt à être fixé ;
- La figure 7 schématise l'ensemble de la figure 6 en position fixée ;
- Les figures 8 et 9 sont des vues en coupe schématique d'une matrice enveloppant partie ou respectivement tout d'une prothèse
- La figure 10 est une variante de la figure 1 quant à la forme du contour périphérique de la feuille.

La matrice dermique acellulaire 1 selon l'invention illustrée sur les figures est notamment destinée à envelopper une prothèse 2 telle qu'une prothèse mammaire, pour former un ensemble 3 destiné à être relié au muscle du grand pectoral lors de la reconstruction mammaire sur un patient.

La matrice 1 présente des dimensions adaptées à l'utilisation finale, d'une part pour recouvrir la prothèse 2 et d'autre part pour correspondre aux dimensions d'accueil de la surface à remplacer par l'ensemble prothèse-matrice.

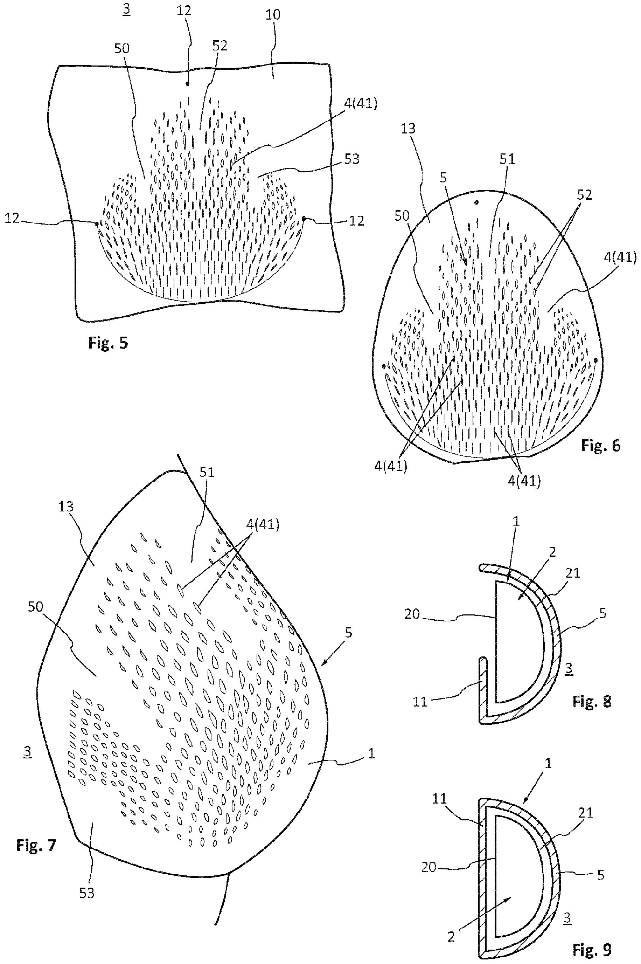

La matrice 1 est de taille adaptée à envelopper une partie seulement de la prothèse (figure 8) ou la totalité de la prothèse (figure 9).

La matrice 1 est généralement de dimensions bien plus grandes que la prothèse et une fois la prothèse enveloppée, sa périphérie est découpée aux dimensions et géométrie souhaitées pour l'ensemble 3.

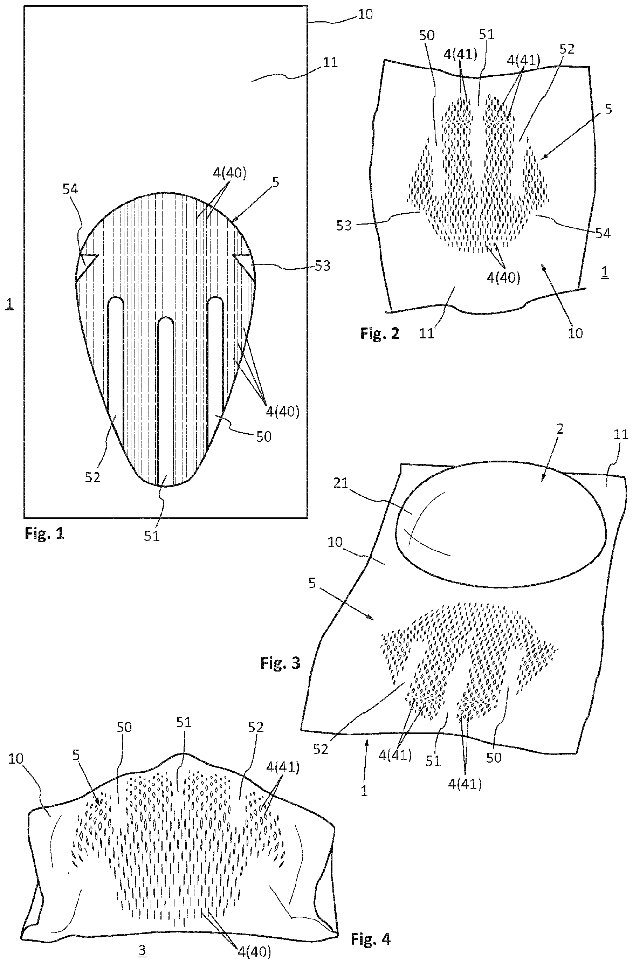

Dans l'exemple représenté, la matrice 1 possède une forme générale à plat qui est rectangulaire (figures 1 à 3).

La matrice 1 est de manière connue faite d'un matériau souple dermique acellulaire, par exemple à partir de derme porcin.

Selon l'invention, la matrice 1 comporte une feuille souple pleine 10 qui comprend une multiplicité d'ouvertures 4 espacées dans une zone localisée 5, les ouvertures 4 étant de forme allongée.

En dehors de la zone localisée perforée 5, la feuille 10 est pleine et présente en particulier une zone pleine d'accueil 11 destinée à l'emplacement de la prothèse 2. La zone pleine 11 peut présenter des dimensions différentes selon la prothèse à accueillir et à recouvrir en totalité ou non.

En regard des figures 2, 8 et 9, la prothèse 2 présente une face dite inférieure 20 qui est destinée à reposer sur/être associée à la zone d'accueil 11, et une face opposée dite supérieure 21 qui est destinée à être recouverte par la zone localisée perforée 5.

Selon l'invention, les ouvertures 4 forment avantageusement des fentes longilignes 40 en position de repos de la matrice lorsque celle-ci ne subit aucun étirement (figure 1). Les fentes sont issues de la découpe localisée de l'épaisseur de la feuille par tous moyens appropriés. En position étirée de la matrice (figures 2 à 7), les fentes 40 sont susceptibles de s'ouvrir pour former des orifices 41 plus larges.

Ainsi, les ouvertures 4 sont aptes à changer de géométrie et de dimensions. Les fentes 40 sont aptes à s'ouvrir par traction exercée sur la matrice.

Selon la traction exercée sur la feuille qui est fonction du relief/de la forme à épouser par la zone perforée 5, les ouvertures 4 restent pour certaines éventuellement sous la forme de fentes 40 et pour les autres s'ouvrent pour former les orifices 41 bien plus larges que les fentes, la section d'ouverture étant variable en fonction de l'emplacement et donc du relief épousé.

Par conséquent, les ouvertures 4 peuvent passer d'une géométrie en forme de fente 40, c'est-à-dire étroite et s'étendant en longueur, à une géométrie expansée/étirée sous la forme d'orifices polygonaux 41, en particulier sous la forme d'orifices de section (sensiblement) en losange.

Pour réaliser la forme polygonale des orifices, le contour de la découpe des fentes est adapté. Certes, la découpe est établie dans une direction principalement longitudinale, mais également dans des directions angulaires aux extrémités de la fente, en particulier à partir de l'affutage adapté des moyens de découpe.

Les fentes 40 sont de préférence toutes identiques. Elles présentent par exemple une longueur de l'ordre de 10 mm. La largeur de la fente correspond à l'épaisseur de la lame des moyens de découpe.

En raison de la fonction élastique de la feuille souple 10, les fentes 40 sont aptes à s'agrandir pour former les orifices 41 de plus grande dimension, essentiellement selon la dimension transversale ou angulaire par rapport à la longueur de la fente. Par exemple, le ratio d'agrandissement des orifices 4 selon la largeur (dimension transversale à la longueur de la fente) est compris entre 2 :1 et 5 :1.

Les ouvertures 4 de la matrice sont donc à grandeur/section variable.

Les ouvertures 4 forment un maillage dans la feuille souple 10, à la manière d'un filet en position d'utilisation de la matrice 1 sur la prothèse 2.

Les ouvertures 4 en position d'utilisation de la matrice 1, c'est-à-dire par recouvrement de la prothèse 2 et par conséquent étirement de la matrice, sont de dimensions/grandeur variables selon leur emplacement dans la zone 5, et donc selon leur localisation sur la prothèse. En effet, la grandeur des ouvertures est fonction de la géométrie/du relief de la prothèse.

Dans l'exemple illustré, la prothèse est une prothèse mammaire de forme bombée. L'étirement des ouvertures 4 sur la partie la plus convexe est bien plus important que sur la périphérie, comme visible sur les figures 4 à 7.

De manière préférée, les orifices 4 sont disposés en colonnes espacées. Les fentes 40 sont alignées en colonnes en présentant leur axe longitudinal dans la direction de la colonne.

De préférence, les fentes 40 sont distribuées en étant équidistantes. Deux colonnes adjacentes sont par exemple séparées (d'une portion pleine) de l'ordre de 2 mm. Deux fentes consécutives d'une même colonne sont par exemple séparées (d'une portion pleine) de 3 mm. Selon l'invention, la zone localisée 5 des ouvertures 4 présente une géométrie et des dimensions adaptées à la destination finale de la matrice 1 (par exemple un sein), et/ou à la forme de la prothèse.

Ici, pour une prothèse mammaire, la zone localisée 5 présente une forme générale ovoïde ou en poire.

Les ouvertures 4 (fentes/orifices) présentent leur longueur s'étendant parallèlement ou sensiblement parallèlement à la plus grande extension de la zone localisée 5, ici au grand axe de la zone localisée 5 de forme ovoïde.

De plus, la zone perforée localisée 5 de préférence des portions pleines plus larges que les espaces pleins de séparation des ouvertures 4. Comme illustré sur les figures, la feuille souple 10 comporte par exemple au moins trois portions pleines 50, 51, et 52 formant des bandes longitudinales partant de la périphérie la plus étroite de la zone localisée 5 sans s'étendre jusqu'à l'extrémité opposée, ainsi que deux portions supplémentaires opposées 53 et 54 rentrantes vers l'intérieur de la zone localisée dans une direction transversale au grand axe et au niveau de la plus grande largeur de la zone ovoïde. Les portions supplémentaires opposées 53 et 54 sont par exemple en forme de triangle.

A titre uniquement illustratif et nullement limitatif, les dimensions associées à la matrice sont les suivantes :
- feuille souple 10 de forme rectangulaire de 400 mm de longueur (pour envelopper la totalité de la prothèse mammaire) ou 200 mm (enveloppement partiel de l'arrière de la prothèse mammaire) par 180 mm de largeur,
- zone localisée 5 des ouvertures 4, de forme ovoïde présentant un grand axe de l'ordre de 190 mm et un petit axe de l'ordre de 115 mm.

La réalisation de l'ensemble 3 prêt à l'utilisation - matrice enveloppant la prothèse - est rapide de mise en oeuvre. Les étapes sont les suivantes :
- la matrice 1 de la figure 1 est plongée à plat dans une solution saline environ cinq minutes ;
- la feuille 10 de la matrice est ensuite posée à plat comme sur la figure 2 et la prothèse 2 est déposée du côté de sa face inférieure 20 sur la zone pleine 11 de la feuille 10 ;
- la feuille 10 est légèrement étirée par les côtés d'extrémité périphériques à la zone perforée 5 afin d'être bien tendue ;
- la zone perforée 5 est alors ramenée sur la face supérieure 21 de la prothèse 2 pour la recouvrir entièrement, comme illustré sur la figure 4 ;
- une étape de couture est opérée à la périphérie 12 de la feuille 10 pour constituer une poche fermée maintenant en place la prothèse 2, l'ensemble 3 est réalisé (figure 5) ;
- de préférence, la périphérie 12 de l'ensemble 3 est découpée selon un contour 13 de géométrie souhaitée (figure 6) quant à sa destination finale.

L'ensemble 3 -matrice dermique acellulaire 1 et prothèse 2 - est prêt à être mis en place et fixé au muscle grand pectoral en étant apte à subir une extension supplémentaire comme schématiquement illustré sur la figure 7.

Dans l'exemple représenté, la matrice 1 possède une forme générale à plat qui est rectangulaire (figures 1 à 3).

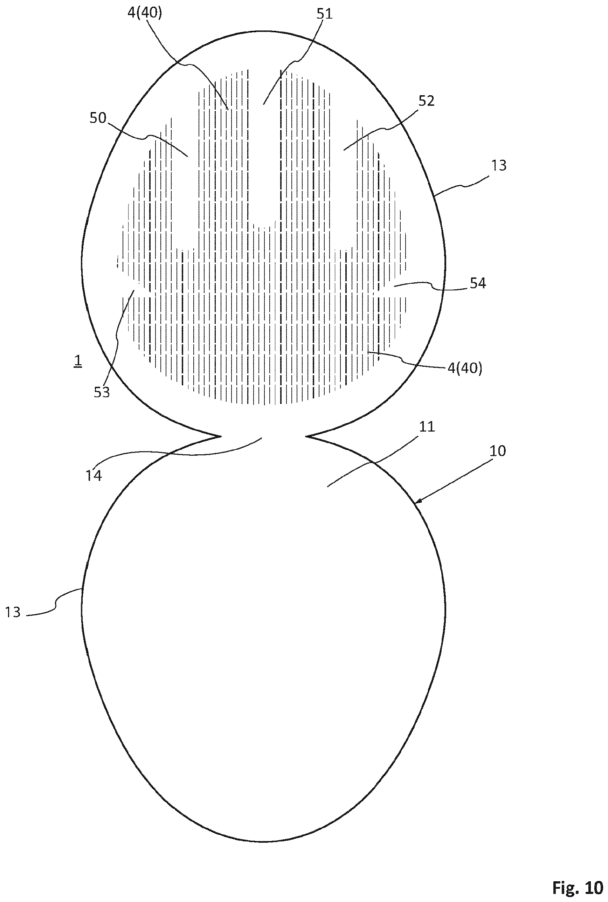

Dans un autre exemple de réalisation de la matrice 1 (figure 10), a matrice 1 possède une forme générale à plat qui est allongée et en forme de deux parties ovoïdes ou en forme de coquillage. Le pourtour périphérique de la feuille 10 présente un contour 13 à ligne arrondie (et non droite comme l'exemple de la feuille rectangulaire) pour s'adapter directement à la forme de la prothèse ayant un contour courbe. La partie dotée de la zone localisée 5 des ouvertures présente une forme en contour 13 symétrique de la partie constituant la zone d'accueil pleine 11, et selon un plan médian transversal à la direction longitudinale de la feuille. De préférence, les deux parties issues de la feuille 10 monolithique sont reliées au niveau d'une zone rétrécie 14 du plan médian de symétrie. Ainsi, la matrice 1 de l'invention et donc l'ensemble 3 permettent d'être étirés de manière équilibrée sur tout son contour 13 et sans pli, grâce à l'adaptation surfacique des ouvertures 4 en fonction du relief épousé. La matrice 1 épouse parfaitement la prothèse 2 recouverte et n'engendre aucun déséquilibre de tension d'étirement en position fixée de l'ensemble 3.

## Revendications

1. Matrice dermique acellulaire (1) comportant une feuille pleine et souple (10) qui est dotée d'une multiplicité d'ouvertures traversantes (4), les ouvertures (4) ont une forme allongée selon au moins une direction, en particulier forment des fentes (40), et sont distribuées en colonnes, **caractérisée en ce que** les ouvertures sont agencées sur la feuille dans une zone localisée (5) formant une surface ovoïde, ladite zone localisée dotée des ouvertures comportant des bandes pleines espacées (50, 51, 52) qui s'étendent dans la direction longitudinale de la surface ovoïde et qui sont plus larges que les espaces pleins de séparation des ouvertures.

2. Matrice dermique acellulaire selon la revendication 1, **caractérisée en ce que** la forme des ouvertures (4) à l'état de repos de la matrice ou à l'état étiré, est oblongue.

3. Matrice dermique acellulaire selon la revendication 1 ou 2, **caractérisée en ce que** les ouvertures (4) sont aptes à s'ouvrir en forme de losange.

4. Matrice dermique acellulaire selon l'une quelconque des revendications précédentes, **caractérisée en ce que** les ouvertures (4) à l'état étiré de la matrice ont une section de grandeur variable.

5. Matrice dermique acellulaire selon l'une quelconque des revendications précédentes, **caractérisée en ce que** les ouvertures sous la forme de fentes présentent une longueur comprise entre 5 et 15 mm, de préférence de l'ordre de 10 mm.

6. Matrice dermique acellulaire selon l'une quelconque des revendications précédentes, **caractérisée en ce que** la forme allongée des ouvertures (4) à l'état de repos de la matrice est telle que le ratio d'expansion de la largeur des ouvertures est d'au moins de 2 pour 1, notamment entre 2 :1 et 5 :1.

7. Matrice dermique acellulaire selon l'une quelconque des revendications précédentes, **caractérisée en ce que** les ouvertures (4) sont séparés d'une distance comprise entre 2 et 5 mm, de préférence de l'ordre de 3 mm pour une même colonne et de l'ordre de 2 mm pour deux colonnes adjacentes.

8. Matrice dermique acellulaire selon l'une quelconque des revendications précédentes, **caractérisée en ce que** les ouvertures (4) sont agencées sur la feuille (10) dans la zone localisée (5), de manière centrale par rapport à l'une des dimensions de la feuille.

9. Matrice dermique acellulaire selon la revendication précédente, **caractérisée en ce que** les bandes pleines longitudinales (50, 51, 52) sont régulièrement espacées, de préférence de largeur comprise entre 5 et 15 mm, de préférence sans s'étendre sur toute la longueur de la zone localisée lorsque ladite zone localisée est oblongue.

10. Matrice dermique acellulaire selon l'une des précédentes revendications, **caractérisée en ce que** la zone localisée (5) dotée d'ouvertures comporte d'une part, les bandes pleines longitudinales (50, 51, 52) s'étendant en longueur dans la direction longitudinale de la feuille, et d'autre part, en d'autres endroits, des portions pleines (53, 54), notamment de forme sensiblement triangulaire, qui sont disposées symétriquement en regard au niveau de la partie la plus large de la zone localisée selon une direction transversale aux zones pleines longitudinales.

11. Matrice dermique acellulaire selon l'une quelconque des revendications précédentes, **caractérisée en ce que** les bandes pleines espacées (50, 51, 52) s'étendent dans la direction longitudinale en partant de l'extrémité la plus étroite de la surface ovoïde sans s'étendre jusqu'à l'extrémité opposée, et de préférence la zone localisée (5) de surface ovoïde comporte des portions pleines (53, 54), notamment de forme sensiblement triangulaire, qui sont disposées symétriquement en regard au niveau de la partie la plus large de la forme ovoïde.

12. Matrice dermique acellulaire selon l'une quelconque des revendications précédentes, **caractérisée en ce que** la feuille est de forme allongée selon une direction longitudinale et présente deux parties opposées selon un plan médian transversal à la direction longitudinale, la première partie (11) est totalement pleine et la seconde partie est pleine et dotée des ouvertures dans la zone localisée (5).

13. Ensemble (3) comportant une matrice dermique acellulaire (1) selon l'une quelconque des revendications précédentes et une prothèse (2), notamment mammaire, qui est recouverte en tout ou partie par la matrice, en particulier les ouvertures (4) étant agencées dans une zone localisée (5) qui correspond à une surface en relief de la prothèse.

## Patentansprüche

1. Azelluläre Hautmatrix (1), umfassend eine massive und flexible Schicht (10), die mit einer Vielzahl von Durchgangsöffnungen (4) versehen ist, wobei die Öffnungen (4) in mindestens einer Richtung eine langgestreckte Form aufweisen, insbesondere Schlitze (40) ausbilden, und in Spalten verteilt sind,
**dadurch gekennzeichnet, dass** die Öffnungen auf der Schicht in einem örtlich begrenzten Bereich (5) angeordnet sind, der eine eiförmige Oberfläche ausbildet, wobei der örtlich begrenzte Bereich, der mit den Öffnungen versehen ist, räumlich getrennte massive Streifen (50, 51, 52) umfasst, die sich in der Längsrichtung der eiförmigen Oberfläche erstrecken und die breiter als die massiven Abstandsräume der Öffnungen sind.

2. Azelluläre Hautmatrix nach Anspruch 1, **dadurch gekennzeichnet, dass** die Form der Öffnungen (4) in dem Ruhezustand der Matrix oder in dem gespannten Zustand länglich ist.

3. Azelluläre Hautmatrix nach Anspruch 1 oder 2,
**dadurch gekennzeichnet, dass** die Öffnungen (4) geeignet sind, um sich rautenförmig zu öffnen.

4. Azelluläre Hautmatrix nach einem der vorstehenden Ansprüche,
**dadurch gekennzeichnet, dass** die Öffnungen (4) in dem gespannten Zustand der Matrix einen Abschnitt variabler Größe aufweisen.

5. Azelluläre Hautmatrix nach einem der vorstehenden Ansprüche,
**dadurch gekennzeichnet, dass** die schlitzförmigen Öffnungen eine Länge zwischen 5 und 15 mm, vorzugsweise in der Größenordnung von 10 mm, vorweisen.

6. Azelluläre Hautmatrix nach einem der vorstehenden Ansprüche,
**dadurch gekennzeichnet, dass** die langgestreckte Form der Öffnungen (4) in dem Ruhezustand der Matrix so ist, dass das Ausdehnungsverhältnis der Breite der Öffnungen mindestens 2 zu 1, insbesondere zwischen 2 : 1 und 5 : 1 beträgt.

7. Azelluläre Hautmatrix nach einem der vorstehenden Ansprüche,
**dadurch gekennzeichnet, dass** die Öffnungen (4) um eine Entfernung zwischen 2 und 5 mm, vorzugsweise in der Größenordnung von 3 mm, für eine gleiche Spalte und in der Größenordnung von 2 mm für zwei benachbarte Spalten, beabstandet sind.

8. Azelluläre Hautmatrix nach einem der vorstehenden Ansprüche,
**dadurch gekennzeichnet, dass** die Öffnungen (4) auf der Schicht (10) in dem örtlich begrenzten Bereich (5) mittig relativ zu einer der Abmessungen der Schicht angeordnet sind.

9. Azelluläre Hautmatrix nach dem vorstehenden Anspruch,
**dadurch gekennzeichnet, dass** die massiven Längsstreifen (50, 51, 52) regelmäßig räumlich getrennt sind, vorzugsweise mit einer Breite zwischen 5 und 15 mm, vorzugsweise ohne sich über die gesamte Länge des örtlich begrenzten Bereichs zu erstrecken, wenn der örtlich begrenzte Bereich länglich ist.

10. Azelluläre Hautmatrix nach einem der vorstehenden Ansprüche,
**dadurch gekennzeichnet, dass** der örtlich begrenzte Bereich (5), der mit Öffnungen versehen ist, einerseits die massiven Längsstreifen (50, 51, 52), die sich in der Längsrichtung der Schicht erstrecken, und andererseits an anderen Stellen massive Abschnitte (53, 54) umfasst, insbesondere von im Wesentlichen dreieckiger Form, die auf der Höhe des breitesten Teils des örtlich begrenzten Bereichs in einer Richtung quer zu den massiven Längsbereichen gegenüberstehend symmetrisch angeordnet sind.

11. Azelluläre Hautmatrix nach einem der vorstehenden Ansprüche,
**dadurch gekennzeichnet, dass** sich die räumlich getrennten massiven Streifen (50, 51, 52) in der Längsrichtung erstrecken, wobei sie an dem schmalsten Ende der eiförmigen Oberfläche beginnen, ohne sich bis zu dem gegenüberliegenden Ende zu erstrecken, und vorzugsweise der örtlich begrenzte Bereich (5) der eiförmigen Oberfläche massive Abschnitte (53, 54) umfasst, insbesondere von im Wesentlichen dreieckiger Form, die auf der Höhe des breitesten Teils der Eiform gegenüberstehend symmetrisch angeordnet sind.

12. Azelluläre Hautmatrix nach einem der vorstehenden Ansprüche,
**dadurch gekennzeichnet, dass** die Schicht von langgestreckter Form in einer Längsrichtung ist und entlang einer Mittelebene quer zu der Längsrichtung zwei gegenüberliegende Teile vorweist, wobei der erste Teil (11) vollständig massiv ist und der zweite Teil massiv ist und in dem örtlich begrenzten Bereich (5) mit Öffnungen versehen ist.

13. Einheit (3), umfassend eine azelluläre Hautmatrix (1) nach einem der vorstehenden Ansprüche und eine Prothese (2), insbesondere für eine Brust, die ganz oder teilweise durch die Matrix bedeckt ist, wobei insbesondere die Öffnungen (4) in einem örtlich begrenzten Bereich (5) angeordnet sind, der einer erhabenen Oberfläche der Prothese entspricht.

## Claims

1. An acellular dermal matrix (1) comprising a solid, flexible sheet (10) which is provided with a multiplicity of through-openings (4), the openings (4) have an elongated shape in at least one direction, in particular form slits (40), and are distributed in columns, **characterized in that** the openings are arranged on the sheet in a localized area (5) forming an ovoid surface, said localized area provided with the openings comprising spaced solid strips (50, 51, 52) which extend in the longitudinal direction of the ovoid surface and which are wider than the solid spaces separating the openings.

2. The acellular dermal matrix according to claim 1, **characterized in that** the shape of the openings (4) in the resting state of the matrix or in the stretched state is oblong.

3. The acellular dermal matrix according to claim 1 or 2, **characterized in that** the openings (4) are capable of opening in a diamond shape.

4. The acellular dermal matrix according to any of the preceding claims,
**characterized in that** the openings (4) in the stretched state of the matrix have a cross-section of variable size.

5. The acellular dermal matrix according to any of the preceding claims,
**characterized in that** the openings in the form of slits have a length of between 5 and 15 mm, preferably of the order of 10 mm.

6. The acellular dermal matrix according to any of the preceding claims,
**characterized in that** the elongated shape of the openings (4) in the resting state of the matrix is such that the expansion ratio of the width of the openings is at least 2:1, in particular between 2:1 and 5:1.

7. The acellular dermal matrix according to any of the preceding claims,
**characterized in that** the openings (4) are separated by a distance of between 2 and 5 mm, preferably of the order of 3 mm for a single column and of the order of 2 mm for two adjacent columns.

8. The acellular dermal matrix according to any of the preceding claims,
**characterized in that** the openings (4) are arranged on the sheet (10) in the localized region (5), centrally with respect to one of the dimensions of the sheet.

9. The acellular dermal matrix according to the preceding claim,
**characterized in that** the longitudinal solid strips (50, 51, 52) are regularly spaced, preferably with a width of between 5 and 15 mm, preferably without extending over the entire length of the localized area when said localized area is oblong.

10. The acellular dermal matrix according to one of the preceding claims,
**characterized in that** the localized area (5) provided with openings comprises, on the one hand, longitudinal solid strips (50, 51, 52) extending lengthwise in the longitudinal direction of the sheet, and on the other hand, at other points, solid portions (53, 54), in particular of substantially triangular shape, which are arranged symmetrically opposite each other at the widest part of the localized area in a direction transverse to the longitudinal solid areas.

11. The acellular dermal matrix according to any of the preceding claims,
**characterized in that** the spaced solid strips (50, 51, 52) extend in the longitudinal direction from the narrowest end of the ovoid surface without extending to the opposite end, and preferably the localized ovoid surface area (5) comprises solid portions (53, 54), in particular of substantially triangular shape, which are arranged symmetrically opposite each other at the widest part of the ovoid shape.

12. The acellular dermal matrix according to any one of the preceding claims,
**characterized in that** the sheet is elongated in a longitudinal direction and has two opposing parts in a median plane transverse to the longitudinal direction, the first part (11) is completely solid and the second part is solid and provided with openings in the localized area (5).

13. An assembly (3) comprising an acellular dermal matrix (1) according to any of the preceding claims and a prosthesis (2), in particular a breast prosthesis, which is covered in whole or in part by the matrix, in particular the openings (4) being arranged in a localized area (5) which corresponds to a raised surface of the prosthesis.
